# EUROPEAN PATENT APPLICATION

(11) **EP 1 834 599 A1**
(43) Date of publication of application: **19.09.2007**
(21) Application number: 05816867.5
(22) Date of filing: 15.12.2005
(51) Int. Cl.: A61B 18/12, A61B 1/00, A61B 17/32

(54) **HOOD WITH EXCISING FUNCTION AND ENDOSCOPE**

(30) Priority: 17.12.2004 JP 2004365457
(71) Applicant: Kyoto University, Kyoto-shi, Kyoto (JP)
(72) Inventor: MIYAMOTO, Shin'ichi c/o Kyoto University Hospital, Sakyo-ku, Kyoto-shi Kyoto 6068507 (JP); NITTA, Takayuki c/o Kyoto University Hospital, Sakyo-ku, Kyoto-shi Kyoto 6068507 (JP)
(74) Representative: Müller-Boré & Partner Patentanwälte
(86) International application number: PCT/JP2005/023033
(87) International publication number: WO 2006/064868

(57) **Abstract**

A cap attachment with excising function attached to the tip of an insert part of an endoscope inserted into the body the cap attachment comprising a cylindrically shaped body part 2 and a plate-shaped excising part projecting from the tip of the body part 2 in the axial direction, and the excising part 4 comprising a tip electrode 6 extending the width direction at the tip thereof. According to this cap attachment with excising function 1, excising operation on organic tissues can be performed easily and accurately.

## Description

### Technical Field

The present invention relates to a cap attachment with a function to excise a portion of organic tissues and an endoscope with the same.

### Background of the Invention

In recent years, for collectively excising a large gastric mucosal lesion, endoscopic submucosal dissection (ESD) has been developed and attracting attention as an alternative to known endoscopic mucosal resection (EMR). In the ESD method, an IT knife (insulated-tip diathermic knife) with a ceramic insulated tip, hooking knife, flex knife and so on are used to incise outside a mark around a lesion around its entire periphery, and then a submucosal layer is dissected with the same knife. The advent of this ESD method has enabled en bloc excising a carcinoma measuring 2 cm or more, which is difficult by the known EMR method.

The known ESD method is generally performed by inserting various kinds of knives into the forceps channel, but poor operability of the knives causes the problem of the necessity for considerable skill and training time in operating them. Therefore, for example, patent document 1 discloses that a constitution in which cleaning water can be supplied from a cap attached at the tip of an endoscope achieves cleaning of blood during treatments and operations, whereby the treatments and operations can be facilitated.
Patent document 1: Japanese Unexamined Patent Publication No. 2003-204921

### Disclosure of the Invention

### Problems to be Solved by the Invention

However, even by the above invention described in patent document 1, treatments and operations are performed with an apparatus inserted into a forceps channel as in conventional cases. Therefore, the problem of knives' poor operability has been still unsolved and has a room for further improvement.

The present invention has been made to solve such problems, and an object of the invention is to provide a cap attachment with excising function and an endoscope comprising the same which can excise organic tissues more easily and accurately.

### Means for Solving the Problems

The object of the present invention is achieved by a cap with excising function which is attached to the tip of an insert part of an endoscope inserted into the body, the cap attachment comprising a cylindrically formed body part and a plate-shaped excising part protruding in the axial direction from the tip of the body part, the excising part comprising tip electrodes extending in the width direction on the tip.

According to this cap attachment with excising function, parts to be excised such as lesion parts can be removed based on the rectilinear operation of the endoscope by operating the endoscope in the protruding direction of the excising part. Since the forceps channel is not necessary for excision operation, it is possible to supply water or insert hemostatic treatment tools and the like through this forceps channel.

The excising part on this cap attachment with excising function preferably comprises a counter electrode for excision opposing the tip electrode on the face rake adjacent to the inner circumferential surface of the body part. In this case, it is more preferable that at least a portion of the face rake is a tapered part so that the excising part becomes thinner toward its tip and the counter electrode for excision is provided in the tapered part. It is more preferable that the excising part comprises a counter electrode for hemostasis opposing the tip electrode on the side opposite to the face rake, and a voltage applied between the tip electrode and the counter electrode for excision, and between the tip electrode and the counter electrode for hemostasis, is switchable. Switching between excising operation and hemostasis operation can be thus quickly performed.

In the cap attachment with excising function mentioned above, a plurality of the tip electrodes can be also disposed and spaced from each other on both sides of the excising part in the width direction. This constitution enables to smoothly perform excising operation by pushing a plurality of the tip electrodes against the parts to be excised and also readily perform hemostasis operation by disposing a bleeding part between electrodes.

In this constitution, the plurality of the tip electrodes are preferably disposed collinearly. The tip electrodes are also preferably disposed in a manner of exposing above the face rake of the excising part adjacent to the inner circumferential surface of the body part to preclude perforations. It is more preferable that at least a portion of the face rake is the tapered part so that the excising part becomes thinner toward the tip. At least one of the plurality of tip electrodes can be provided with a projection for incision protruding in the axial direction.

The purpose of the present invention is achieved by an endoscope in which an insert part which is inserted into the body is provided, the cap attachment with excising function mentioned above being provided at the tip of the insert part.

### Effect of the Invention

According to the present invention, a cap attachment with excising function which can easily and accurately perform excising operation on organic tissues, and an endoscope comprising this cap attachment with excising function can be provided.

### Brief Description of the Drawings

[Fig. 1] Fig. 1 is a plan view of a cap attachment with excising function according to a first embodiment of the present invention.
[Fig. 2] Fig. 2 is a cross-sectional view taken along line A-A in Fig. 1.
[Fig. 3] Fig. 3 is a diagram which shows an example of wiring in the cap attachment with excising function shown in Fig. 1.
[Fig. 4] Fig. 4 is a drawing for illustrating the excising method of a lesion part and others by using the cap attachment with excising function.
[Fig. 5] Fig. 5 is a perspective view showing a cap attachment with excising function according to a second embodiment of the present invention.
[Fig. 6] Fig. 6 is a plan view of the cap attachment with excising function shown in Fig. 5.
[Fig.7] Fig. 7 is a cross-sectional view taken along line B-B in Fig. 6.
[Fig. 8] Fig. 8 is a waveform chart showing an example of a coagulation wave of a high-frequency current.
[Fig. 9] Fig. 9 is a front view showing a variation of the cap attachment with excising function shown in Fig. 5.
[Fig. 10] Fig. 10 is a plan view of another variation of the cap attachment with excising function shown in Fig. 5.

### Description of the Numerals

1, 101 Cap attachment with excising function
2 Body part
4 Excising part
6, 6a, 6b Tip electrodes
6c, 6d Projections for incision
8 Counter electrode for excision
10 Counter electrode for hemostasis
14 High-frequency generating apparatus
16 Switch box
20 Endoscope
24 Forceps channel
41 Face rake
42 Tapered part
43 Opposite face

### Best Mode for Carrying out the Invention

Embodiments of the present invention will be described below with reference to accompanying drawings.

### (First embodiment)

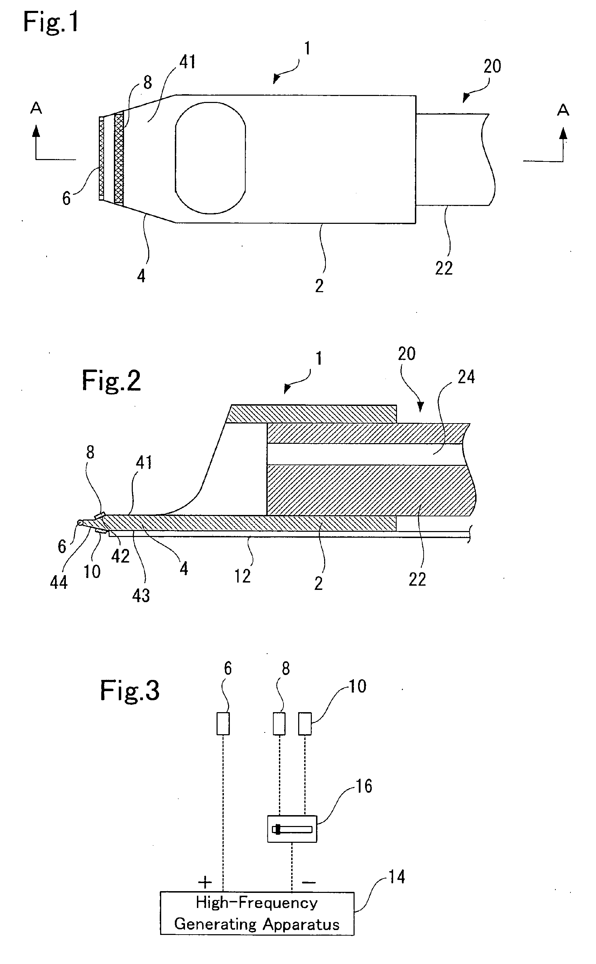

Fig. 1 is a plan view showing a cap attachment with excising function according to the first embodiment of the present invention.
Fig. 2 is a cross-sectional view taken along line A-A in Fig. 1.

As shown in Figs. 1 and 2, the cap attachment with excising function 1 has a cylindrically formed body part 2 and an excising part 4 protruding from the tip of this body part 2.

The body part 2 is formed to have a shape and dimension corresponding to the tip of an insert part 22 so that it is attachable to the insert part 22 of an endoscope 20 inserted into the body, and is attached in a manner of covering a portion around the tip of the insert part 22, but partially protruding from the tip of the insert part 22. The endoscope 20 has a forceps channel 24 through which water can be supplied and which various kinds of apparatuses can be inserted, and as well as known endoscopes, a lighting system comprising a light guide, a lighting lens and other components, and an image pickup system comprising a CCD camera and other components (not shown). The cap attachment with excising function 1 can be fixed by a tape or the like so that the tip of the excising part 4 easily comes into the field of vision of the endoscope 20.

The excising part 4 is formed in the shape of a plate and is provided to extend from a portion of the end face of the body part 2 in the axial direction. As shown in Fig. 2, the face adjacent to the inner circumferential surface of the body part 2 at the excising part 4 (that is, the axis side face of the body part 2) serves as a face rake 41 for excising a lesion part and the like. In addition, in this embodiment, the inner circumferential surface of the body part 2 and the face rake of the excising part 4 are smoothly connected, but a step may be formed therebetween.

A portion of the face rake 41 has a tapered part 42 becoming thinner from the side of the body part 2 toward the tip side. In this embodiment, the surface of the tapered part 42 forms a straight line in side view. For example, the surface may be formed to have a curved line whose rate of changing of thickness gradually becomes smaller from the end face to the tip of the body part 2.

In this embodiment, the excising part 4 is formed in such a manner that its cross section is approximately constant in the width direction (the direction perpendicular to the axial direction in Fig. 1). For example, the excising part 4 may be also formed into a curved plate in such a manner that its central part is concave in the width direction with respect to both sides of the face rake 41. Materials of the excising part 4 are not especially limited, but are preferably those with high heat resistance, processability and strength. Examples include fluorine-based resins such as polytetrafluoroethylene (PTFE), tetrafluoroethylene-ethylene copolymers (ETFE), tetrafluoroethylene-hexafluoropropylene copolymers (FEP), tetrafluoroethylene-perfluoroalkylvinyl ether copolymers (PFA) and the like. In view of transparency and processability, PFA is especially preferred. The excising part 4 may be integrally formed with the body part 2, or may be constituted of a component different from the body part 2.

In this embodiment, the tip edge of the excising part 4 is formed in a straight-line in plan view, and the tip electrodes 6 are formed in the width direction along this tip edge. The tip edge of the excising part 4 may be formed in the shape of an arc in such a manner that its central part protrudes in plan view.

On the face rake 41 in the excising part 4, the counter electrode for excision 8 is provided in a manner of opposing the tip electrodes 6 and being spaced (e.g., about 1 mm) therefrom. In this embodiment, the surface area of the counter electrode for excision 8 is formed to be greater than that of the tip electrodes 6.

In contrast, an back face 43 on the reverse side of the face rake 41 in the excising part 4 is formed to be the tapered part 44 which becomes thinner from the side of the body part 2 toward the tip, and the counter electrode for hemostasis 10 is provided in a manner of opposing the tip electrodes 6 and being spaced (for example, about 1 to 2 mm) therefrom. As in the counter electrode for excision 8, the surface area of the counter electrode for hemostasis 10 is also formed to be greater than that of the tip electrodes 6. The tip electrode 6, the counter electrode for excision 8 and the counter electrode for hemostasis 10 can be constituted of, for example, conductive materials such as copper, silver and the like, and are preferably treated with an insulation coating of fluorine resins and the like. The width length of the tip electrode 6 is, for example, about 0.3 to 1 mm, and the width length of the counter electrode for excision 8 and the counter electrode for hemostasis 10 is, for example, about 1 to 3 mm.

The tip electrode 6, the counter electrode for excision 8 and the counter electrode for hemostasis 10 are connected to the high-frequency generating apparatus 14 via a coated wire 12 fixed on the outer surface side of the excising part 4 and the body part 2 in the axial direction. More specifically, as shown in Fig. 3, the tip electrode 6 is connected to the anode side of the high-frequency generating apparatus 14, while the counter electrode for excision 8 and the counter electrode for hemostasis 10 are connected to the cathode side of the high-frequency generating apparatus 14 via the switch box 16. The switch box 16 can switch a voltage applied between the tip electrode 6 and the counter electrode for excision 8, and between the tip electrode 6 and the counter electrode for hemostasis 10 by switching operation.

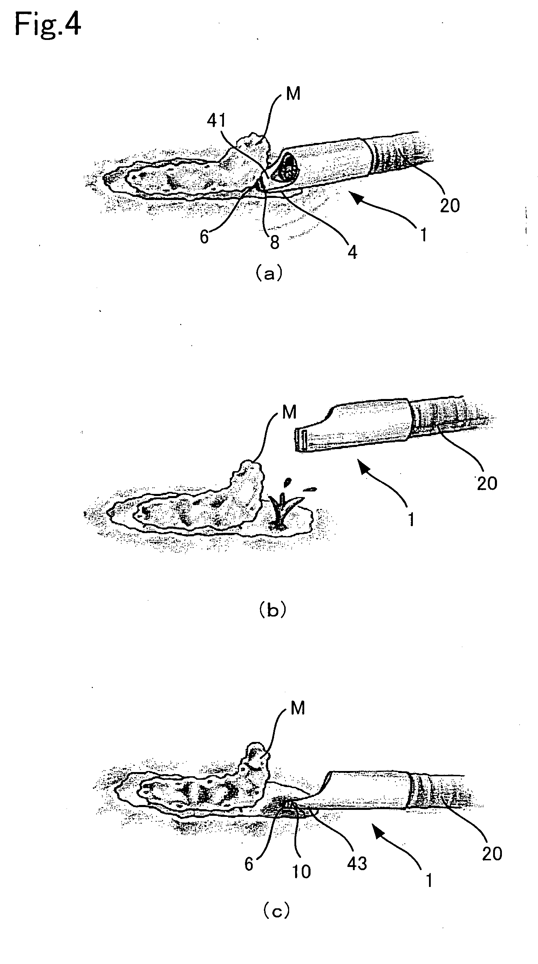

The excising method of a lesion part and the like by using the cap attachment with excising function 1 having the above constitution will be described now with reference to Fig. 4, taking endoscopic submucosal dissection of the submucosal layer for example. Firstly, as in conventional case, the lesion area is marked, and then incision is made outside the mark around its entire periphery with an IT knife. Secondly, the cap attachment with excising function 1 of this embodiment is attached to the tip of the insert part 22 on the endoscope 20, and this endoscope 20 is inserted into a body cavity. In a state that a voltage is applied between the tip electrode 6 and the counter electrode for excision 8 by operating the switch box 16, the cap attachment with excising function 1 is operated while monitoring the area around the tip of the endoscope 20, and, as shown in Fig. 4 (a), a mucosal layer M containing the lesion part is excised with the face rake 41 side of the excising part 4 at the depth of the submucosal layer. When the submucosal layer comes between and into contact with the tip electrode 6 and the counter electrode for excision 8, a high-frequency current flows to the submucosal layer between the tip electrode 6 and the counter electrode for excision 8, and this electrical energy enables to perform submucosal layer dissection smoothly. Since the excising part 4 is provided in a manner of protruding in the axial direction of the cap attachment with excising function 1, submucosal layer dissection by the excising part 4 is performed basically by moving the cap attachment with excising function 1 linearly by inserting the endoscope, which can be performed without special skills, thereby achieving accurate excising operation readily in a short period of time.

In this embodiment, since the surface area of the counter electrode for excision 8 is formed to be greater than that of the tip electrode 6, the current density is higher on the side of the tip electrode 6, whereby submucosal layer dissection can be performed more smoothly. Moreover, the counter electrode for excision 8 is provided on the tapered part 42 of the face rake 41, which makes the submucosal layer on the side of the face rake 41 more conductive, and makes insertion of the excising part 4 into the submucosal layer easy.

As shown in Fig. 4 (b), in case of bleeding during submucosal layer dissection, a voltage is applied between the tip electrode 6 and the counter electrode for hemostasis 10 by operating the switch box 16. As shown in Fig. 4 (C), hemostasis is carried out by pressing the bleeding part with the back face 43 opposite to the excising part 4 to coagulate blood by a current flow. Thus, the cap attachment with excising function 1 in this embodiment can instantly switch between an incision mode using the face rake 41 side of the excising part 4 and a coagulation mode using the back face 43 side, by operating the switch box 16, whereby a series of operations can be performed quickly and accurately.

In this embodiment, the tip electrode 6, the counter electrode for excision 8 and the counter electrode for hemostasis 10 are provided on the excising part 4 of the cap attachment with excising function 1, and application of a voltage is switchable between the tip electrode 6 and the counter electrode for excision 8, and between the tip electrode 6 and the counter electrode for hemostasis 10, but the counter electrode for hemostasis 10 may not be provided so that a voltage can be applied only between the electrode 6 and the counter electrode for excision 8. Even in this constitution, excising operation can be readily performed without inserting various knives through the forceps channel of the endoscopes as in conventional examples. Since excising operation can be performed in a state that the forceps channel 24 is freely available, when hemostasis is necessary, water can be supplied and hemostatic treatment tools can be inserted through the forceps channel 24, whereby the hemostatic operation, as well as excising operation, can be facilitated. In this constitution, both the tip electrode 6 and the counter electrode for excision 8 may be disposed on the face rake 41 of the excising part 4.

The constitution may be of mono polar type in which the excising part 4 comprises only the tip electrode 6 as the cap attachment with excising function 1. In this case, a counter electrode plate can be disposed outside the body of the patient and a high-frequency current is applied between this the counter electrode plate and the tip electrode 6, whereby tissue excising operation can be performed readily and accurately, as well as in the bipolar type.

### (Second embodiment)

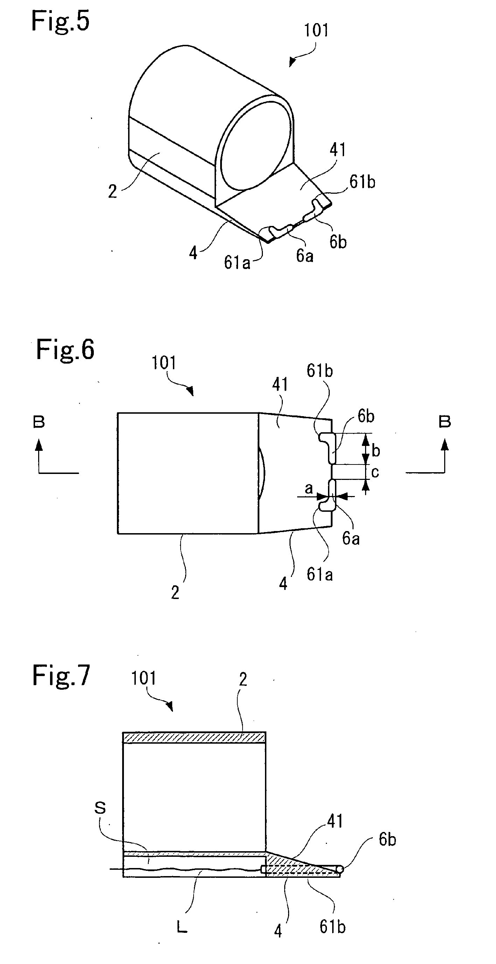

Fig. 5 is a perspective view of the cap attachment with excising function according to the second embodiment of the present invention. Fig. 6 is a plan view of the cap attachment with excising function. Fig. 7 is a cross-sectional view taken along line B-B in Fig. 6. The cap attachment with excising function in this embodiment, as well as the first embodiment, can be also attached to the tip of the insert part on the endoscope, but the endoscope is not shown in Figs. 5 to 7. In Figs. 5 to 7, components of the cap attachment with excising function similar to those in the first embodiment shown in Figs. 1 to 4 are given identical numerals to abbreviate detailed description.

In the cap attachment with excising function 101 in this embodiment, the entire face rake 41 of the excising part 4 is a tapered part (for example, tapered angle: 15 degrees) whose thickness becomes thinner from the body part 2 side toward the tip side, and a pair of insert holes 61 a, 61 b extending from the tip side of the face rake 41 toward the body part 2 parallel to the axial direction are formed on the excising part 4. One ends of hook members made of metal are inserted into the insert holes 61a, 61 b, respectively. The hook members are connected to the anode and cathode, respectively, of the high-frequency generating apparatus (not shown) via the lead wires L. The lead wires L extend to the back side of the body part 2 through a containing space S formed in a lower part of the body part 2. a resin (not shown) having electrical insulation such as a fluorine resin or the like is sealed in the containing space S.

Meanwhile, the other end sides of the hook members are exposed above the face rake 41, and constitute the tip electrodes 6a, 6b. The two tip electrodes 6a, 6b are disposed on each side of the excising part 4, respectively, and spaced from each other in the width direction. The two tip electrodes 6a, 6b in this embodiment are in the form of straight bars, and are disposed on the same straight line extending in the width direction of the excising part 4, but their arrangement is not necessarily limited to this. For example, the two tip electrodes may be in the shape of arcs and disposed on opposite sides across the axis of the body part 2 in the width direction. Materials of the excising part 4 and the tip electrodes 6a, 6b can be, for example, the same as those of the excising part 4 and the tip electrodes 6 in the first embodiment.

In Fig. 6, the width a of the tip electrodes 6a, 6b is preferably about 0.5 to 1.5 mm, and the length b is preferably about 4 to 6 mm. An interval c between the tip electrodes 6a, 6b is suitably about 1 to 2 mm to prevent discharge and enable smooth excising operation. The distal end side of the tip electrodes 6a, 6b (that is, the vicinity of the insert holes 61a, 61 b) may be coated with a fluorine resin and the like to prevent deposition of tissues and the like during excising operation.

The two tip electrodes 6a, 6b of the cap attachment with excising function 101 in this embodiment are disposed on both sides of the cap attachment with excising function 101 in the width direction so that the tip of the excising part 4 can be thin, whereby the visibility of the endoscope can be improved and excising operation can be readily performed by pressing in the axial direction.

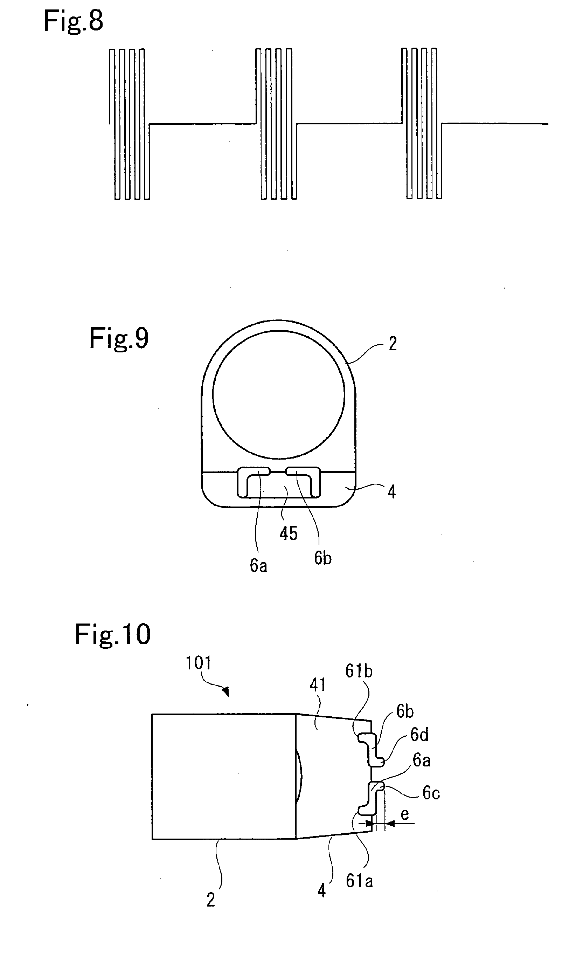

That is, as shown in Fig. 8, in a state that the tip of the excising part 4 is pressed against the tissues such as submucosal layer and the like, an intermittent wave (burst wave) of a high-frequency current, which is utilized as a coagulation wave for general electrosurgical knives, is applied between the tip electrodes 6a, 6b and pressure is applied in the direction of travel of the endoscope, whereby excising operation can be smoothly performed with almost no bleeding or tissue injuries and other problems. Even in the case of bleeding during excising operation, hemostatic treatment can be quickly performed without changing the waveform of a high-frequency current applied to the tip electrodes 6a, 6b by locating a bleeding part between the tip electrodes 6a, 6b and pressing the bleeding part slightly against the electrodes. The electric power of the burst wave during excising and hemostasis is, for example, 15 to 20 W, which achieves operation performed with low power consumption.

In the constitution of this embodiment, as well as in the first embodiment, the forceps channel is freely available. Therefore, water supply and inserting and extracting other treatment tools can be freely performed by utilizing this forceps channel.

In this embodiment, the entire face rake 41 of the excising part 4 is the tapered part to make the tip of the excising part 4 thin and facilitate excising operation with the tip electrodes 6a, 6b, but the tapered part of the face rake 41 may be formed only on a portion of the tip side. Alternatively, a constitution in which no tapered part is provided on the face rake 41 of the excising part 4 is also possible. For example, as shown in Fig. 9, a notch 45 may be formed at the tip of the excising part 4 having no tapered part on the face rake 41 and the tip electrodes 6a, 6b may be accommodated in this notch part 45. The tip electrodes 6a, 6b shown in Fig. 9 are disposed collinearly and spaced from each other in the width direction of the excising part 4 by disposing the hook members along the tip face of the excising part 4, and the tip electrodes 6a, 6b are exposed above the face rake 41. This constitution can greatly reduce the risk of perforation.

In this embodiment, as shown in Fig. 10, the tip electrodes 6a, 6b may be provided with projections for incision 6c, 6d by bending their tip parts in the axial direction of the cap attachment with excising function 101 or by other processes. According to this constitution, a current can be applied between a pair of the projections for incision 6c, 6d by applying a current to the tip electrodes 6a, 6b. Therefore, for example, excision of tissues can be performed by pressing the projections for incision 6c, 6d against tissues such as mucous membrane to incise the lesion site around its entire periphery, and then utilizing the tip electrodes 6a, 6b, and a series of operations can be therefore efficiently performed. In the constitution shown in Fig. 10, the projecting width e of the projections for incision 6c, 6d is preferably about 1 to 2 mm. The projections for incision 6c, 6d may be also provided on only one of, instead both of, the pair of the tip electrodes 6a, 6b. Also in this case, application of a current between the projection for incision provided on one of the tip electrodes and the other tip electrode enables to perform incision operation.

### (Examples)

The present invention will be described in more detail with reference to Examples. However, the present invention is not limited to these examples.

A gastric mucosa resection experiment was performed to evaluate the operability, degree of bleeding, and hemostatic control performance of the cap attachment with excising function 101 in the second embodiment shown in Figs. 5 to 7 by using a two-month-old pig (25 kg) according to following procedure.

1. A mixture of 2 ml of atropine sulfate (0.5 mg/ml, Tanabe Seiyaku Co., Ltd), 6 ml of Ketalar 50 for animal use (50 mg/ml, Sankyo Co., Ltd.), 4 ml of Stresnil for animal use (40 mg/ml, Sankyo Co., Ltd.) was injected as a premedication into the muscle on the back of the pig, and then inhalation anesthesia of Fluothane (Takeda Healthcare Company) was started through a nasotracheal cannula (0.32 L/min) immediately thereafter. Simultaneously, a route was secured in auricular veins with a 23 G indwelling needle to supplement intravenous anesthesia of Thiamylal Sodium (Nichi-lko Pharmaceutical Co., Ltd.) via a bypass depending on the pig's condition of anesthesia (2.5 mg/kg at a time).

2. The electrocardiogram and oxygen saturation were monitored, and stable anesthesia was confirmed. Thereafter, an over-tube (flexible over-tube, Sumitomo Bakelite Co., Ltd.) was indwelled inside the oral cavity of the pig, and the endoscope was inserted therethrough.

3. A 2-cm piece of paper was placed as a hypothetical lesion on the greater curvature in the lower gastric corpus by using a forceps. After marking around the lesion with a hooking knife (Olympus, KD-620LR), the piece of paper was removed. Subsequently, a 10% glycerol solution (Chugai Pharmaceutical Co., Ltd.) containing 0.01 mg/ml of epinephrine (Daiichi Pharmaceutical. Co., Ltd.) was locally injected around the mark to form a sufficient protrusion under the mucous membrane. A round incision was made outside the mark on the mucous membrane around its entire periphery with a needle knife (Olympus KD-1 L) and an IT knife (Insulated-tip diathermic knife, Olympus, KD-610L).

4. Subsequently, the 10% glycerol solution containing epinephrine was locally injected sufficiently from the incised layer into the submucosal layer, and then the endoscope was extracted. The cap attachment with excising function 101 was fixed at the tip of the endoscope with a tape. The visibility around the tip of the excising part 4 was very good on the monitor.

5. The endoscope was inserted again, and the cap attachment knife was thrusted against the submucosal layer in the direct vision to proceed incision in a forced coagulation mode (ICC-200, ERBE, Germany) at 15 W. Incision was performed very smoothly with little bleeding. Even when bleeding occurred, it was readily stopped only by fixing the bleeding point between the electrodes and applying a current. The operation was basically pressing only, and the operation after the dissection proceeded to some degree and the entire knife was inserted into submucosal layer could be fully performed in direct vision and was extremely safe. The dissection was finished in about 20 minutes, and the dissected face was smooth.

The dimension of the excised sample was 5 cm. Although the estimated time to complete the dissection by a known method was 2 hours at the shortest, the experiment demonstrated that the cap attachment with excising function of the present invention can shorten the time required for the procedure and improve safety simultaneously. Furthermore, excision of esophageal mucosa was attempted by the same method. Because the esophagus has a wall that is thinner and a lumen that is narrower than those of the stomach, respectively, it has apparently more risks of perforation than in the stomach by known methods. However, the cap attachment with excising function of the present invention had no problem of operability or safety even in the esophagus, and showed the strong possibility of its application to organs in which it has been thought to be difficult to en bloc excise a large portion of the mucosa, such as the esophagus, large intestine and other organs.

### Industrial applicability

The cap attachment with excising function of the present invention can be suitably utilized in excising a lesion on the mucosa in the stomach, and is applicable to not only gastric carcinomas, but also to mucosal lesions in the esophagus, large intestine and other organs. The cap attachment with excising function of the present invention is also suitably attached to an endoscope which may be a flexible scope for observing the gastrointestinal tracts and the like, or a rigid scope such as a laparoscope, cystoscope and the like.

## Claims

1. A cap attachment with excising function which is attached to the tip of an insert part of an endoscope inserted into the body,
the cap attachment comprising a cylindrically formed body part
and a plate-shaped excising part protruding in the axial direction from the tip of the body part,
the excising part comprising tip electrodes extending in the width direction on the tip.

2. A cap attachment with excising function according to claim 1, wherein the excising part comprises a face rake adjacent to the inner circumferential surface of the body part, and the face rake is provided with a counter electrode for excision opposing the tip electrode.

3. A cap attachment with excising function according to claim 2, wherein at least a portion of the face rake is a tapered part so that the excising part becomes thinner toward the tip,
and the counter electrode for excision is provided on the tapered part.

4. A cap attachment with excising function according to claim 2, wherein a counter electrode for hemostasis opposing the tip electrode is provided on the side opposite to the face rake of the excising part, and a voltage applied between the tip electrode and the counter electrodes for excision, and between the tip electrode and the counter electrode for hemostasis, is switchable.

5. A cap attachment with excising function according to claim 1, wherein a plurality of the tip electrodes are disposed and spaced from each other on both sides of the excising part in the width direction.

6. A cap attachment with excising function according to claim 5, wherein a plurality of the tip electrodes are disposed collinearly.

7. A cap attachment with excising function according to claim 5, wherein the excising part comprises a face rake adjacent to the inner circumferential surface of the body part, the tip electrodes being disposed in a manner of exposing above the face rake.

8. A cap attachment with excising function according to claim 7, wherein at least a portion of the face rake is a tapered part so that the excising part becomes thinner toward the tip.

9. A cap attachment with excising function according to claim 5, wherein a projection for incision protruding in the axial direction is provided on at least one of a plurality of the tip electrodes.

10. An endoscope comprising an insert part which is inserted into the body and a cap attachment with excising function according to claim 1 at the tip of the insert part.
